# EUROPEAN PATENT APPLICATION

(11) **EP 3 087 983 A1**
(43) Date of publication of application: **02.11.2016**
(21) Application number: 14874918.7
(22) Date of filing: 25.12.2014
(51) Int. Cl.: A61K 31/196, A61K 47/02, A61K 47/32, A61K 47/34, A61P 27/04, A61P 43/00

(54) **EYE DROPS FOR TREATING DRY EYE**

(30) Priority: 25.12.2013 JP 2013267514
(71) Applicant: LTT Bio-Pharma Co., Ltd., Minato-ku Tokyo 105-0022 (JP)
(72) Inventor: TAKAHASHI, Yuji, Tokyo 103-8330 (JP); MIZUSHIMA, Tohru, Tokyo 105-0022 (JP)
(74) Representative: Webster, Jeremy Mark
(86) International application number: PCT/JP2014/084261
(87) International publication number: WO 2015/099019

(57) **Abstract**

The present invention aims to provide an ophthalmic solution for the treatment of dry eye which is used to inhibit apoptosis caused by tear hyperosmolarity. The present invention relates to an ophthalmic solution for the treatment of dry eye, containing diclofenac or a pharmaceutically acceptable salt thereof, the ophthalmic solution being used to inhibit apoptosis caused by tear hyperosmolarity.

## Description

### TECHNICAL FIELD

The present invention relates to an ophthalmic solution for the treatment of dry eye.

### BACKGROUND ART

Dry eye is a multifactorial chronic disease of tears and corneal and conjunctival epithelia that results in symptoms of eye discomfort and visual disturbance. Dry eye is a major eye disease from which 10 to 20% of adults suffer in the Europe, the U.S., and Japan. The number of patients with dry eye is increasing due to the increased amount of time spent in front of displays, dry air from air conditioning, wearing of contact lenses, and other factors.

Dry eye is caused by decreased tear secretion by the lacrimal gland, or increased moisture evaporation of tears associated with lipid and mucin abnormalities, which results in a decreased amount of tears. The decrease in the amount of tears causes chronic irritation or inflammation on the corneal and conjunctival surfaces, leading to a decrease in the quality of life of patients. In order to reduce such inflammation and treat dry eye, steroid drugs have been used. Steroid drugs, however, are not considered to be safe enough to use and tend to cause adverse reactions. It is known that nonsteroidal anti-inflammatory drugs (NSAIDs) such as diclofenac and bromfenac can be used instead of steroid drugs to reduce inflammation. Patent Literature 1 discloses an anti-inflammatory ophthalmic solution that contains diclofenac as an active ingredient.

Known methods for treating dry eye are based on anti-inflammatory activity. However, methods for the treatment of dry eye which are based on non-anti-inflammatory mechanism of action have been needed because it is thought that, in addition to inflammatory causes, dry eye can also be associated with aging, hormonal changes, environmental causes, autoimmunity, and other issues.

### CITATION LIST

### PATENT LITERATURE

Patent Literature 1: JP S58-174310 A

### SUMMARY OF INVENTION

### TECHNICAL PROBLEM

The present invention aims to provide an ophthalmic solution for the treatment of dry eye which is used to inhibit apoptosis caused by tear hyperosmolarity.

### SOLUTION TO PROBLEM

The present invention relates to an ophthalmic solution for the treatment of dry eye, containing diclofenac or a pharmaceutically acceptable salt thereof, the ophthalmic solution being used to inhibit apoptosis caused by tear hyperosmolarity.

The diclofenac or pharmaceutically acceptable salt thereof is preferably at a concentration of 0.01 to 0.7% by weight/volume of the ophthalmic solution.

The pharmaceutically acceptable salt of diclofenac is preferably diclofenac sodium.

The ophthalmic solution preferably further contains polysorbate 80, borax, or polyvinylpyrrolidone.

### ADVANTAGEOUS EFFECTS OF INVENTION

Since the ophthalmic solution for the treatment of dry eye according to the present invention contains diclofenac or a pharmaceutically acceptable salt thereof, the ophthalmic solution can inhibit apoptosis caused by tear hyperosmolarity and thereby effectively treat dry eye.

### BRIEF DESCRIPTION OF DRAWINGS

Fig. 1A is a view showing the results of Example 1 and Comparative Example 1.
Fig. 1B is another view showing the results of Example 1 and Comparative Example 1.
Fig. 2A is a view showing the results of Example 2 and Comparative Example 2.
Fig. 2B is another view showing the results of Example 2 and Comparative Example 2.
Fig. 3A is a view showing the results of Example 3 and Comparative Example 3.
Fig. 3B is another view showing the results of Example 3 and Comparative Example 3.
Fig. 3C is yet another view showing the results of Example 3 and Comparative Example 3.
Fig. 4A is a view showing the results of Example 4 and Comparative Example 4.
Fig. 4B is another view showing the results of Example 4 and Comparative Example 4.
Fig. 4C is yet another view showing the results of Example 4 and Comparative Example 4.
Fig. 5A is a view showing the results of Example 5 and Comparative Example 5.
Fig. 5B is another view showing the results of Example 5 and Comparative Example 5.
Fig. 5C is yet another view showing the results of Example 5 and Comparative Example 5.
Fig. 5D is yet another view showing the results of Example 5 and Comparative Example 5.
Fig. 5E is yet another view showing the results of Example 5 and Comparative Example 5.
Fig. 6 is a view showing the results of Example 6 and Comparative Example 6.
Fig. 7 is a view showing the results of Example 7 and Comparative Example 7.
Fig. 8 is a view showing the results of Example 8 and Comparative Example 8.
Fig. 9 is a view showing the results of Example 9 and Comparative Example 9.
Fig. 10 is a view showing the results of Example 10 and Comparative Example 10.

### DESCRIPTION OF EMBODIMENTS

The present invention relates an ophthalmic solution for the treatment of dry eye, containing diclofenac or a pharmaceutically acceptable salt thereof, the ophthalmic solution being used to inhibit apoptosis caused by tear hyperosmolarity.

The concentration of the diclofenac or pharmaceutically acceptable salt thereof in the ophthalmic solution is preferably 0.01 to 0.7% by weight/volume, more preferably 0.05 to 0.5% by weight/volume. A concentration of lower than 0.01% by weight/volume tends to lead to reduced treatment effects. With a concentration of higher than 0.7% by weight/volume, the composition tends to be difficult to prepare.

Examples of the pharmaceutically acceptable salt of diclofenac include diclofenac sodium and diclofenac potassium.

The pH of the ophthalmic solution is preferably 6.0 to 8.5, more preferably 7.0 to 8.0. A pH of lower than 6.0 tends to fail to relieve eye irritation, while a pH of higher than 8.5 is out of the physiological pH range.

The osmotic pressure ratio of the ophthalmic solution is preferably 0.9 to 1.4. The osmotic pressure ratio as used herein refers to the osmotic pressure ratio relative to physiological saline.

The ophthalmic solution of the present invention containing diclofenac or a pharmaceutically acceptable salt thereof may further contain a buffer, isotonizing agent, preservative, thickener, solubilizer, and detergent.

Examples of the buffer include combinations of phosphoric acid and salts of phosphoric acid, a combination of boric acid and borax, and combinations of organic acids and salts of organic acids. Among these, the combination of boric acid and borax is preferred. The buffer content in the ophthalmic solution is preferably 0.01 to 10% by weight/volume, more preferably 0.1 to 3% by weight/volume. With a buffer content of lower than 0.01% by weight/volume, the resulting effect of the present invention tends to be less sufficient. A buffer content of higher than 10% by weight/volume tends to cause eye irritation.

Examples of the isotonizing agent include sugars such as glucose, propylene glycol, glycerol, sodium chloride, potassium chloride, and sugar alcohols such as mannitol, sorbitol, and xylitol. Among these, sodium chloride or potassium chloride is preferred. The isotonizing agent content in the ophthalmic solution is preferably 0.01 to 10% by weight/volume, more preferably 0.1 to 3% by weight/volume.

Examples of the preservative include invert soaps such as benzalkonium chloride, benzethonium chloride, and chlorhexidine gluconate; parabens such as methylparaben, ethylparaben, propylparaben, and butylparaben; and alcohols such as chlorobutanol, phenylethyl alcohol, and benzyl alcohol. Among these, chlorobutanol is preferred. The preservative content in the ophthalmic solution is preferably 0.001 to 0.5% by weight/volume.

Examples of the thickener include polyvinylpyrrolidone, methylcellulose, hydroxypropyl methylcellulose, and hydroxypropyl cellulose. Among these, polyvinylpyrrolidone is preferred.

Examples of the solubilizer include polysorbate 80 (polyoxyethylenesorbitan monooleate, trade name: Tween 80), polyoxyethyleneoxystearic acid triglyceride, polyethylene glycol, and α- or β-cyclodextrin. Among these, polysorbate 80 is preferred.

The ophthalmic solution may contain a calcium salt or a magnesium salt in order to relieve eye irritation. Examples of these salts include calcium salts such as calcium pantothenate, calcium chloride, calcium propionate, calcium acetate, calcium lactate, and calcium gluconate, and corresponding magnesium salts. Among these, calcium pantothenate, calcium chloride, and magnesium chloride are preferred.

The ophthalmic solution of the present invention preferably contains polysorbate 80, borax, or polyvinylpyrrolidone, among the above components that can be combined, to enhance the effect of inhibiting apoptosis caused by hyperosmolarity.

In order to enhance the effect of inhibiting apoptosis caused by hyperosmolarity, the concentration of polysorbate 80 in the ophthalmic solution is preferably 0.1 to 5.0% by weight/volume, more preferably 0.3 to 3.0% by weight/volume.

In order to enhance the effect of inhibiting apoptosis caused by hyperosmolarity, the concentration of borax in the ophthalmic solution is preferably 0.1 to 20.0% by weight/volume, more preferably 0.3 to 15.0% by weight/volume.

In order to enhance the effect of inhibiting apoptosis caused by hyperosmolarity, the concentration of polyvinylpyrrolidone in the ophthalmic solution is preferably 1.0 to 15.0% by weight/volume, more preferably 2.0 to 10.0% by weight/volume.

The present invention relates to an ophthalmic solution for the treatment of dry eye which is used to inhibit apoptosis caused by tear hyperosmolarity, among other ophthalmic solutions. Usually, dry eye is associated with a decrease in the amount of tears, resulting in increased tear osmolarity. This increase puts the cells under osmotic stress, where efflux of water from the cells occurs, resulting in shrinkage of the cells. The cells respond to the stress by taking up extracellular ions such as sodium ions, which increases the intracellular ionic strength, thereby causing apoptosis of the cells. Ocular tissues in which apoptosis can occur include the cornea, conjunctiva, and lacrimal gland. More specifically, cells in which apoptosis can occur include corneal epithelial cells, conjunctival epithelial cells, and lacrimal-gland cells.

The diclofenac used in the ophthalmic solution of the present invention inhibits apoptosis even with hyperosmotic tears, by promoting the expression and nuclear translocation of the nuclear factor of activated T-cells 5 (NFAT5) gene. NFAT5 gene products activate the betaine/GABA transporter-1 (BGT-1) gene and other genes. This allows the cells to respond to the osmotic stress by taking up organic osmolytes which do not affect the ionic strength. Thus, an increase in the ionic strength in the cells can be avoided, and therefore it is considered that apoptosis is inhibited even with hyperosmotic tears. The ophthalmic solution of the present invention exhibits the effect of inhibiting apoptosis in the cornea, conjunctiva, and lacrimal gland caused by tear hyperosmolarity. More specifically, the ophthalmic solution of the present invention exhibits the effect of inhibiting apoptosis in corneal epithelial cells, conjunctival epithelial cells, and lacrimal-gland cells.

In use of the ophthalmic solution of the present invention, preferably one to three drops, more preferably one to two drops, are instilled in each eye per application. Moreover, the volume of drops instilled in each eye per application is preferably 10 to 300 µL, more preferably 20 to 200 µL, still more preferably 30 to 100 µL. The dosage interval of the ophthalmic solution of the present invention is preferably one to six times daily, more preferably one to three times daily.

### EXAMPLES

The present invention will be described in more detail with reference to examples. The examples, however, are not intended to limit the scope of the present invention.

### (Example 1) Effect of reducing cytotoxicity under hyperosmotic conditions

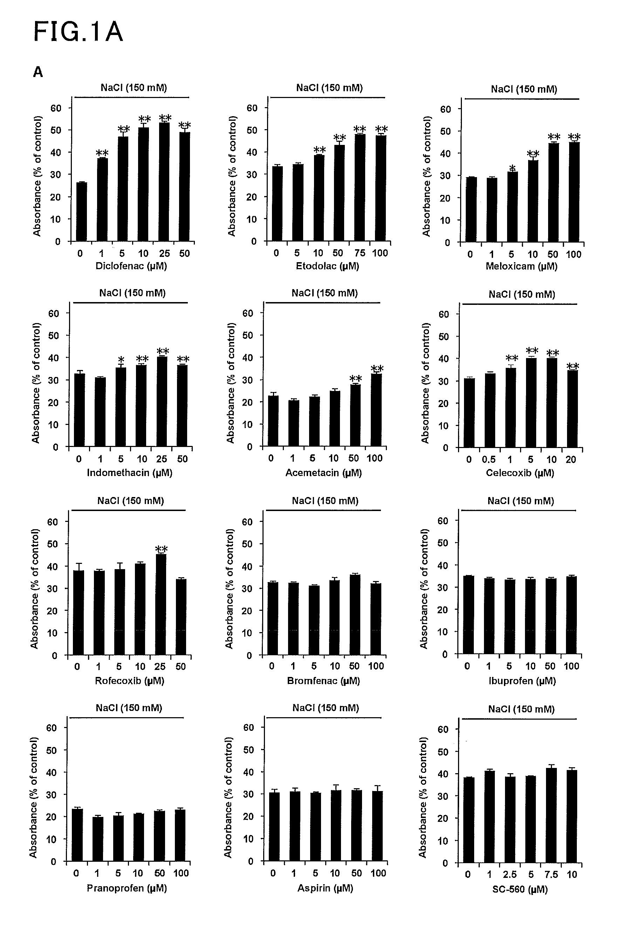

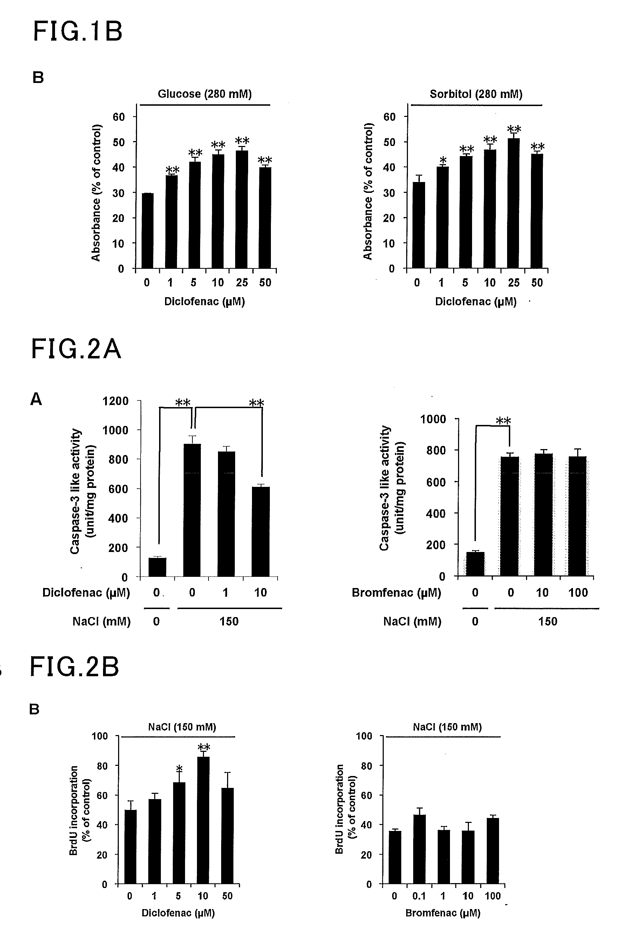

Human corneal epithelial cells (HCE cells) were incubated in hyperosmotic media containing diclofenac. The hyperosmotic conditions of each medium were created by 150 mM NaCl, 280 mM glucose, or 280 mM sorbitol. The number of viable cells was measured by MTT assay, and the absorbance was calculated relative to the control (under isotonic conditions). The results are shown in Fig. 1A and Fig. 1B. The values are represented as average ± S.D. (n = 3), with a single asterisk (*) indicating P < 0.05 and a double asterisk (**) indicating P < 0.01.

### (Comparative Example 1)

The same procedure as in Example 1 was followed, except that other nonsteroidal anti-inflammatory drugs (NSAIDs) were used in place of diclofenac. The results are shown in Fig. 1A.

The results demonstrated that, among other nonsteroidal anti-inflammatory drugs (NSAIDs), especially diclofenac was highly effective in reducing cytotoxicity under hyperosmotic conditions.

### (Example 2) Effect of inhibiting apoptosis under hyperosmotic conditions and cell proliferative effect

HCE cells were incubated in hyperosmotic media containing diclofenac and 150 mM NaCl. After six hours of incubation, the caspase-3-like activity was measured using a fluorescent peptide substrate. The results are shown in Fig. 2A. Furthermore, after 12 hours of incubation, cell proliferation was assessed by BrdU incorporation assay, and the absorbance data were expressed as relative values to that of the control (under isotonic conditions). The results are shown in Fig. 2B. The values are represented as average ± S.D. (n = 3), with a single asterisk (*) indicating P < 0.05 and a double asterisk (**) indicating P < 0.01.

### (Comparative Example 2)

The same procedure as in Example 2 was followed, except that bromfenac was used in place of diclofenac. The results are shown in Fig. 2A and Fig. 2B.

The results demonstrated that diclofenac was effective in inhibiting apoptosis under hyperosmotic conditions and further exhibited a cell proliferative effect.

### (Example 3) Independence from COX-inhibition

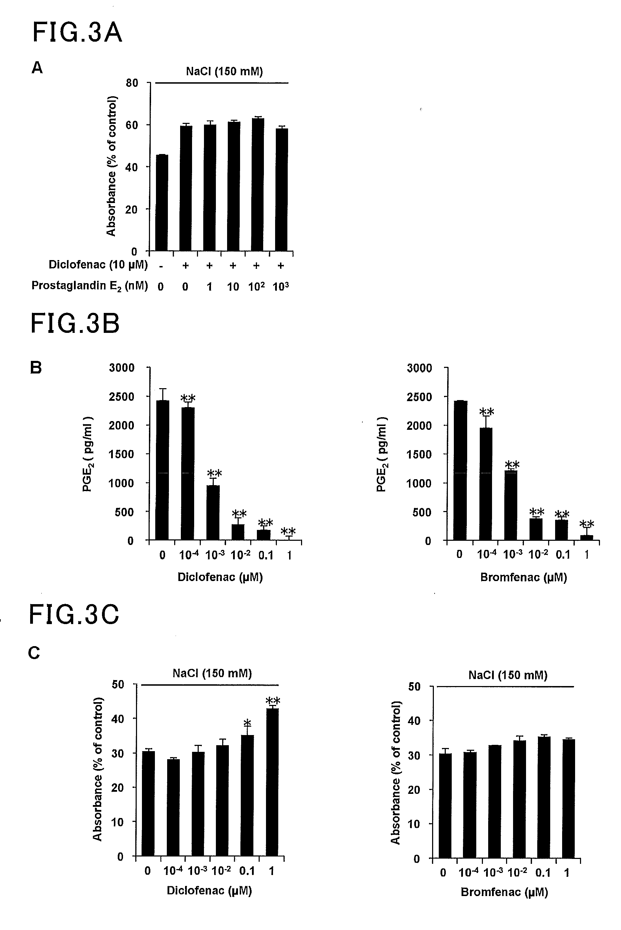

HCE cells were incubated in hyperosmotic media containing diclofenac, 150 mM NaCl, and/or PGE₂ for 24 hours. The number of viable cells was measured by MTT assay, and the absorbance data were expressed as relative values to that of the control (under isotonic conditions). The results are shown in Fig. 3A and Fig. 3C.

Separately, HCE cells were pre-incubated in media containing diclofenac for 30 minutes, and then incubated in media containing 10 µM arachidonic acid and diclofenac. The arachidonic acid was added to induce PGE₂. The amount of PGE₂ contained in each culture was measured by EIA. The results are shown in Fig. 3B. The values are represented as average ± S.D. (n = 3), with a single asterisk (*) indicating P < 0.05 and a double asterisk (**) indicating P < 0.01.

### (Comparative Example 3)

The same procedure as in Example 3 was followed, except that bromfenac was used in place of diclofenac. The results are shown in Figs. 3A to 3C.

The number of viable cells did not change with PGE₂ as shown in Fig. 3A. The concentration of diclofenac required to reduce PGE₂ was 0.1 nM as shown in Figs. 3B and 3C, whereas the concentration required to reduce cytotoxicity caused by hyperosmolarity was 100 nM as shown in Fig. 3C. Since inflammation is generally known to be caused by increased production of prostaglandin E₂ (PGE₂) through cyclooxygenase (COX), the results in Figs. 3A to 3C demonstrated that the effect produced by diclofenac is independent of the effect of inhibiting COX to lower the PGE₂ expression level.

### (Example 4) Effects of enhancing expression and nuclear translocation of NFAT5

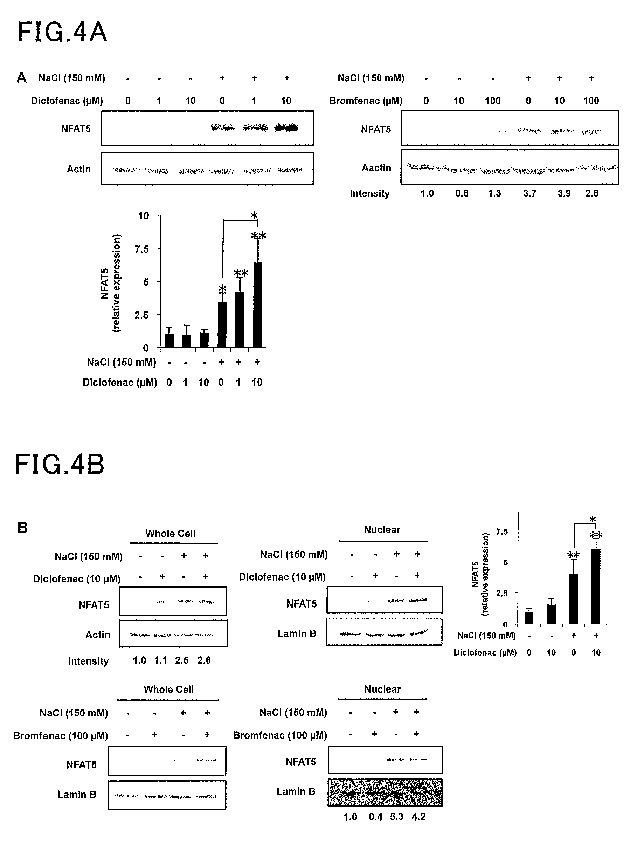

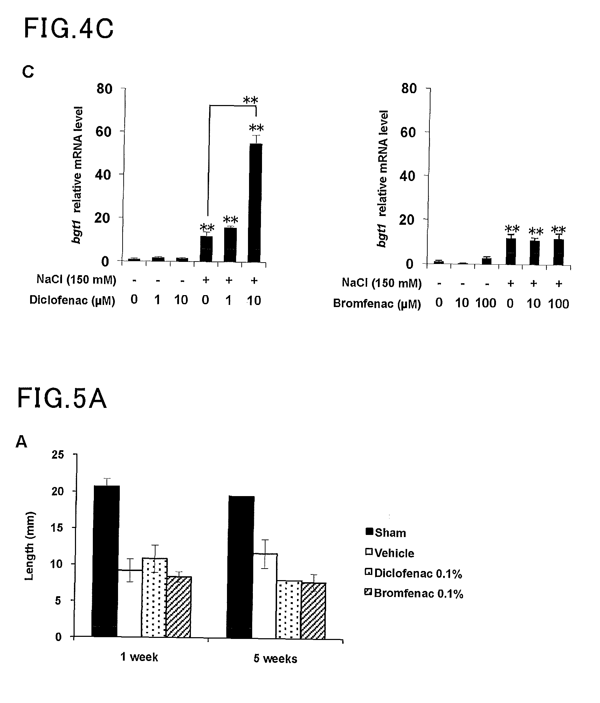

HCE cells were incubated in hyperosmotic media containing diclofenac and 150 mM NaCl for six hours (Fig. 4A and Fig. 4C) or for one hour (Fig. 4B). Each whole cell homogenate (Fig. 4A and Fig. 4B) or nuclear extract (Fig. 4B) was analyzed by immunoblotting using an anti-NFAT5, anti-actin, or anti-lamin B antibody. The intensity of the NFAT5 band was measured and expressed as relative values to that of the control (under isotonic conditions without diclofenac) (Fig. 4A and Fig. 4B). The relative bgt1 mRNA expression level was measured by real-time RT-PCR, and the values normalized by the actin expression level are expressed as relative values to that of the control (under isotonic conditions without diclofenac) (Fig. 4C). The values are represented as average ± S.D. (n = 3), with a single asterisk (*) indicating P < 0.05 and a double asterisk (**) indicating P < 0.01.

### (Comparative Example 4)

The same procedure as in Example 4 was followed, except that bromfenac was used in place of diclofenac. The results are shown in Figs. 4A to 4C.

The results demonstrated that diclofenac inhibited apoptosis under hyperosmotic conditions by the effects of enhancing the expression and nuclear translocation of NFAT5.

### (Example 5) Effect of treating corneal surface disorders in rat

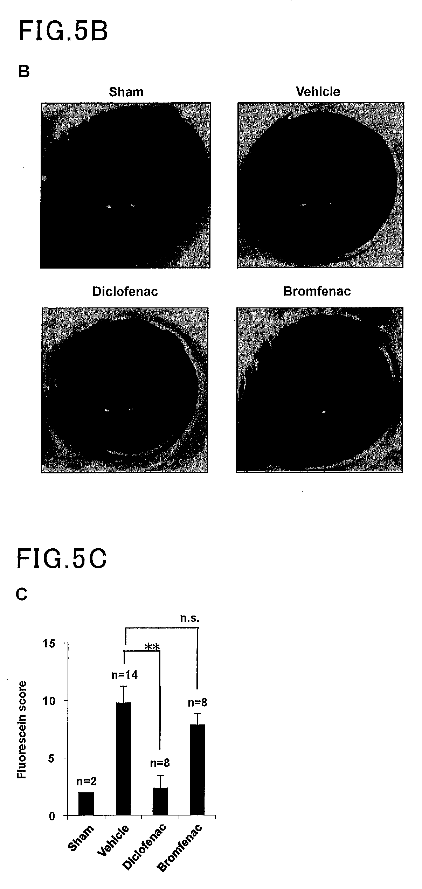

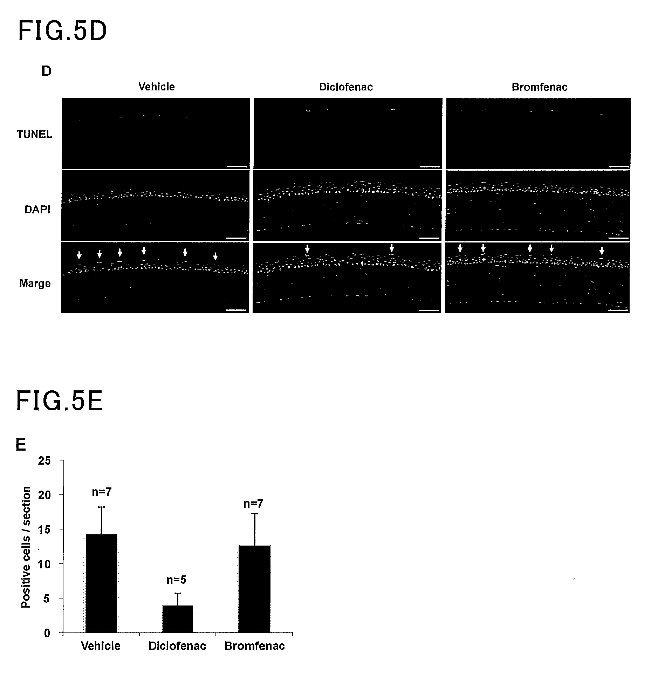

The lacrimal gland of a rat was removed to prepare a dry eye model. One to five weeks after the lacrimal gland removal, an ophthalmic solution (5 µl) containing diclofenac (0.1%, 3.1 mM) was administered three times a day. The amount of tears was measured by the cotton thread test. The results are shown in Fig. 5A. The images of the cornea stained with fluorescein are shown in Fig. 5B. The fluorescein scores calculated are shown in Fig. 5C. Five weeks after the lacrimal gland removal, ocular tissue sections were prepared and subjected to TUNEL assay and DAPI staining. The results are shown in Fig. 5D (scale bar = 50 µm). The numbers of TUNEL positive cells are shown in Fig. 5E. The values are represented as average ± S.E.M., with a single asterisk (*) indicating P < 0.01 and n.s. meaning "not significant".

### (Comparative Example 5)

The same procedure as in Example 5 was followed, except that bromfenac was used in place of diclofenac. The results are shown in Figs. 5A to 5E.

The results demonstrated that diclofenac exhibited the effect of treating corneal surface disorders in the dry eye model.

### (Example 6) Combined use of diclofenac and polysorbate 80

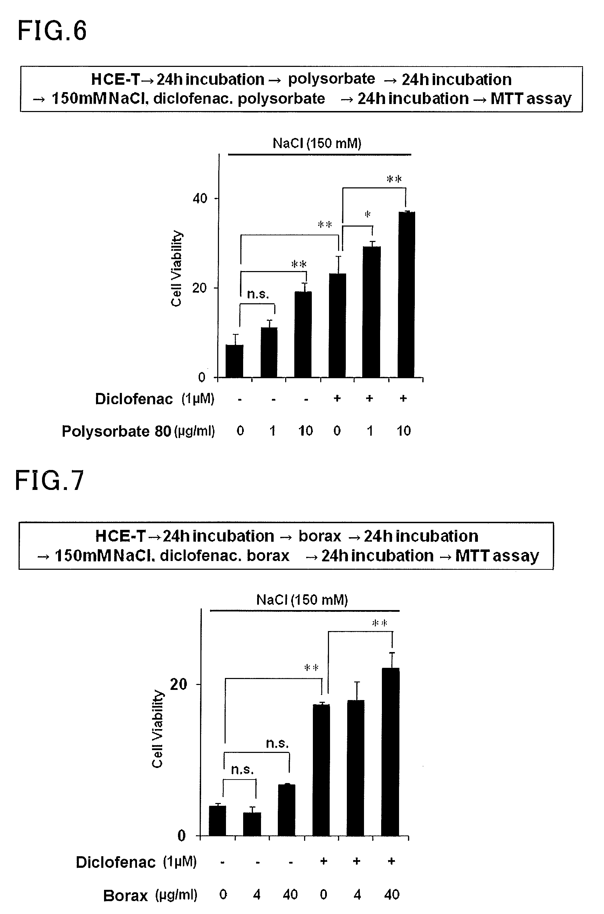

HCE cells were pre-incubated in media containing 0 µg/ml, 1 µg/ml, or 10 µg/ml polysorbate 80 for 24 hours. The HCE cells were further incubated for 24 hours in hyperosmotic media containing 1 µM diclofenac, 150 mM NaCl, and polysorbate 80 at the same concentration as that in the pre-incubation. The number of viable cells was measured by MTT assay, and the results are shown in Fig. 6.

### (Comparative Example 6)

The same procedure as in Example 6 was followed, except that diclofenac was not used. The results are shown in Fig. 6.

The results demonstrated that the combined use of polysorbate 80 with diclofenac enhanced the effect of diclofenac.

### (Example 7) Combined use of diclofenac and borax

HCE cells were pre-incubated in media containing 0 µg/ml, 4 µg/ml, or 40 µg/ml borax for 24 hours. The HCE cells were further incubated for 24 hours in hyperosmotic media containing 1 µM diclofenac, 150 mM NaCl, and borax at the same concentration as that in the pre-incubation. The number of viable cells was measured by MTT assay, and the results are shown in Fig. 7.

### (Comparative Example 7)

The same procedure as in Example 7 was followed, except that diclofenac was not used. The results are shown in Fig. 7.

The results demonstrated that the combined use of borax with diclofenac enhanced the effect of diclofenac.

### (Example 8) Combined use of diclofenac and povidone

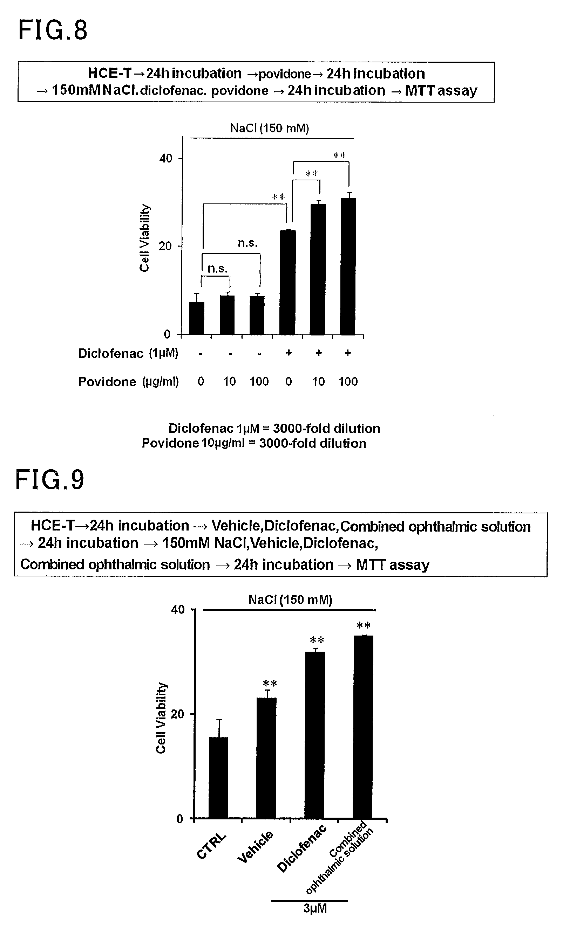

HCE cells were pre-incubated in media containing 0 µg/ml, 10 µg/ml, or 100 µg/ml povidone for 24 hours. The HCE cells were further incubated for 24 hours in hyperosmotic media containing 1 µM diclofenac, 150 mM NaCl, and povidone at the same concentration as that in the pre-incubation. The number of viable cells was measured by MTT assay, and the results are shown in Fig. 8.

### (Comparative Example 8)

The same procedure as in Example 8 was followed, except that diclofenac was not used. The results are shown in Fig. 8.

The results demonstrated that the combined use of povidone with diclofenac enhanced the effect of diclofenac.

### (Example 9) Effect of ophthalmic solution containing borax, boric acid, chlorobutanol, povidone, and polysorbate 80 in combination (hereinafter, referred to as combined ophthalmic solution)

HCE cells were pre-incubated in a medium containing 1 µg/ml combined ophthalmic solution for 24 hours. The HCE cells were further incubated for 24 hours in a hyperosmotic medium containing 3 µM combined ophthalmic solution and 150 mM NaCl. The number of viable cells was measured by MTT assay. Additionally, the same procedure was followed, but using diclofenac instead of the combined ophthalmic solution. The results are shown in Fig. 9. The combined ophthalmic solution is an ophthalmic solution having the composition shown in Formulation 3.

### (Comparative Example 9)

The same procedure as in Example 9 was followed, except that diclofenac or the combined ophthalmic solution was not used (CTRL), or a buffer was used (Vehicle). The results are shown in Fig. 9.

The results demonstrated that the combined use of borax, boric acid, chlorobutanol, povidone, and polysorbate 80 with diclofenac enhanced the effect of diclofenac.

### (Example 10)

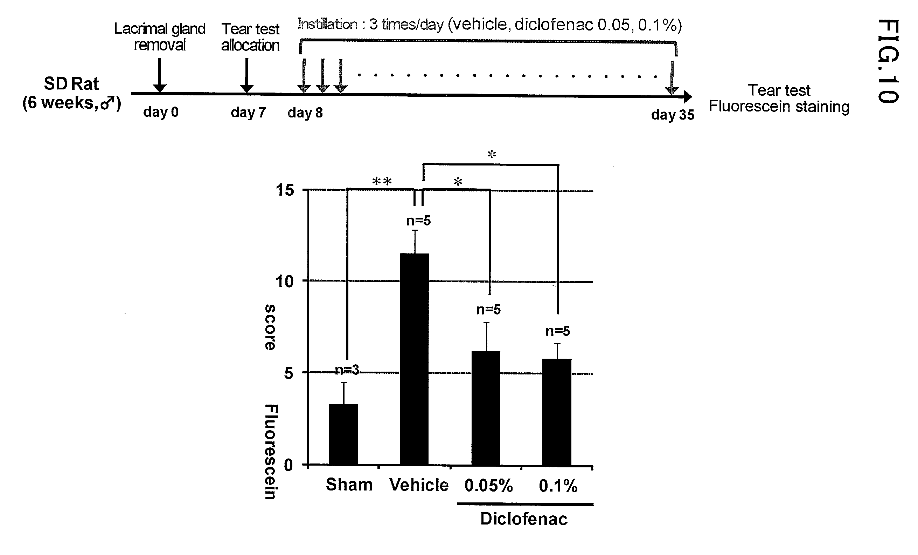

The lacrimal gland of a rat was removed to prepare a dry eye model. One week after the lacrimal gland removal, an ophthalmic solution (5 µl) containing diclofenac (0.05%, 1.55 mM; or 0.1%, 3.1 mM) was administered three times a day. After four weeks from the start of the administration, the tears were subjected to fluorescein staining, and the fluorescein score was calculated. The results are shown in Fig. 10.

### (Comparative Example 10)

The same procedure as in Example 10 was followed, except that a buffer was used (Vehicle) in place of diclofenac. The results are shown in Fig. 10.

The results demonstrated that corneal surface disorders in the dry eye model are treatable with 0.05% diclofenac.

### (Formulation 1)

Diclofenac sodium (100 mg), borax (573 mg), boric acid (868 mg), sodium chloride (290 mg), and β-cyclodextrin (100 mg) are dissolved in distilled water (about 80 ml). Then, calcium lactate (150 mg) is added to and dissolved in the solution. The resulting solution is diluted with distilled water to 100 ml and filtered, whereby an ophthalmic solution is prepared.

### (Formulation 2)

Diclofenac sodium (100 mg), NaH₂PO₄ (anhydrous) (200 mg), Na₂HPO₄ (anhydrous) (710 mg), sodium chloride (300 mg), and β-cyclodextrin (1000 mg) are dissolved in distilled water (about 80 ml). Then, calcium pantothenate (150 mg) is added to and dissolved in the solution. The resulting solution is diluted with distilled water to 100 ml and filtered, whereby an ophthalmic solution is prepared.

### (Formulation 3)

Diclofenac sodium (100 mg), borax (450 mg), boric acid (1500 mg), chlorobutanol (500 mg), polyvinylpyrrolidone K25 (3000 mg), and polysorbate 80 (Tween 80) (500 mg) are dissolved in sterile purified water to give a total amount of 100 ml. Thus, an ophthalmic solution is prepared.

## Claims

1. An ophthalmic solution for the treatment of dry eye, comprising diclofenac or a pharmaceutically acceptable salt thereof, the ophthalmic solution being used to inhibit apoptosis caused by tear hyperosmolarity.

2. The ophthalmic solution according to claim 1,
wherein the diclofenac or pharmaceutically acceptable salt thereof is present at a concentration of 0.01 to 0.7% by weight/volume of the ophthalmic solution.

3. The ophthalmic solution according to claim 1 or 2,
wherein the pharmaceutically acceptable salt of diclofenac is diclofenac sodium.

4. The ophthalmic solution according to any one of claims 1 to 3, further comprising polysorbate 80, borax, or polyvinylpyrrolidone.
